# EUROPÄISCHE PATENTSCHRIFT

(11) **EP 1 495 782 B1**
(45) Veröffentlichungstag und Bekanntmachung des Hinweises auf die Patenterteilung: **21.12.2005**
(21) Anmeldenummer: 03015403.3
(22) Anmeldetag: 08.07.2003
(51) Int. Cl.: A61M 37/00, A45D 34/04

(54) **Vorrichtung zum Aufbringen einer Tätowierung oder von permanentem Make-up**
Tattooing and permanent make-up device
Système de tatouage et de maquillage permanent

(43) Veröffentlichungstag der Anmeldung: 12.01.2005
(73) Patentinhaber: MediUm-TECH Medizingeräte GmbH, 14167 Berlin (DE)
(72) Erfinder: Frister, Tilo, 13053 Berlin (DE)
(74) Vertreter: Bittner, Thomas L.

(56) Entgegenhaltungen:
- EP-A- 0 374 355
- DE-U- 20 012 369
- DE-U- 29 919 199
- DE-U- 29 923 931
- US-A- 5 776 158

## Beschreibung

Die Erfindung liegt auf dem Gebiet von Vorrichtungen zum Aufbringen einer Tätowierung und/oder von permanentem Make-up.

Solche Vorrichtungen werden in der Regel als Handgeräte ausgeführt, die von Bedienpersonal zum Aufbringen eines Farbstoffs für die Tätowierung und/oder das permanente Make-up im Bereich der Hautoberfläche verwendet werden. Ein solches Handgerät ist beispielsweise aus der Druckschrift DE 299 19 199.0 bekannt. Das bekannte Handgerät umfaßt ein Griffstück, einen Nadelantrieb, eine Nadel und eine Nadeldüse, wobei mindestens zwei lösbar miteinander verbundene Module vorgesehen sind und das eine der beiden Module als wiederverwendbares Grundmodul mit integriertem Nadelantrieb ausgebildet ist. Das andere der beiden Module ist ein sterilisiere Einwegmodul, indem alle durch die Köperflüssigkeiten eines Kunden infizierbaren Bestandteile des Handgeräts integriert sind. Auf diese Weise wird das Handgerät in Form von zwei Modulen zur Verfügung gestellt, von denen eines, nämlich das Einwegmodul, nach der Benutzung ausgetauscht werden kann, während das andere Modul, welches den Nadelantrieb umfaßt, wieder verwendet wird. Mit Hilfe des Einwegmoduls werden die hygenischen Bedingungen beim Aufbringen einer Tätowierung und/oder von permanetem Make-up verbessert, da alle Teile ausgetauscht werden, die potentiell durch die bei der Behandlung austretende Körperflüssigkeit des Kunden infiziert werden können. Es wird so vermieden, daß das gesamte Handgerät ausgetauscht werden muß.

Aus der Druckschrift EP 0 374 355 A2 ist ein Modul zur Vewendung in einer Tätowiervorrichtung bekannt. Das Modul verfügt vor einer Nutzung über an beiden Enden aufgesteckte Schutzkappen, die vor einer ersten Benutzung des Moduls abgenommen werden können. Das Modul kann dann an ein Handstück gekoppelt werden, mit dem eine Antriebsbewegung für eine in dem Modul gelagerte Nadel erzeugt wird. Die Nadel ist in einem Nadelschaft gelagert, an dem sich in dem Modul Verlängerungsbauteile zum Einkoppeln einer linearen Antriebsbewegung auf den Nadelschaft anschließen.

In der Druckschrift US 5,776,158 ist eine Vorrichtung zum Tätowieren beschrieben. Zum Erzeugen einer linearen Vorwärts- und Rückwärtsbewegung einer in einem Nadelschaft gelagerten Nadel ist ein Motor mit einer drehenden Antriebswelle vorgesehen. Eine der Nadel zugewandte Endfläche der Antriebswelle ist angeschrägt, so daß beim Drehen der Antriebswelle ein Kippbauteil um eine Kippachse repetierend verschwenkt wird. Auf diese Weise wird die Drehbewegung der Antriebswelle in eine Vorwärts-/Rückwärtsbewegung eines an dem Kippbauteil gebildeten Kopplungsendes umgewandelt. An das Kopplungsende werden Kopplungsbauteile angekoppelt, um die lineare Bewegung aufzunehmen und in den Nadelschaft zum Bewegen der Nadel beim Tätowieren einzleiten.

Aufgabe der Erfindung ist es, eine verbesserte Vorrichtung zum Aufbringen einer Tätowierung oder von permantem Make-up anzugeben, bei der die Nutzungseigenschaften der Vorrichtung optimiert sind.

Diese Aufgabe wird erfindungsgemäß durch eine Vorrichtung nach dem unabhängigen Anspruch 1 gelöst.

Die Erfindung umfaßt den Gedanken, bei einer Vorrichtung zum Aufbringen einer Tätowierung oder von permanentem Make-up, welche ein an einem Handstück lösbar angeordnetes Einwegmodul, eine Nadel, die mit einem Ende in einem Nadelschaft gehalten wird, die bewegbar in einer an dem Einwegmodul gebildeten Nadelführung gelagert ist und die mit einem anderen Ende durch eine Nadeldüsenöffnung zum Ausbringen eines Farbstoffs verläuft und eine an den Nadelschaft zum Vor- und Zurückbewegen der Nadel gekoppelten und von mehreren Bauteilen gebildeten Antriebsmechanismus umfaßt, wobei die mehreren Bauteile des Antriebsmechanismus einen Antrieb und Kopplungsmittel zum Koppeln des Antriebs an den Nadelschaft aufweisen, vorzusehen, daß zumindest ein Teil der mehreren Bauteile des Antriebsmechanismus in dem Einwegmodul angeordnet ist und zusammen mit dem Einwegmodul von dem Handstück abnehmbar ist.

Der wesentliche Vorteil, welcher mit der Erfindung gegenüber dem Stand der Technik erreicht wird, besteht darin, daß beim Abnehmen des Einwegmoduls von dem Handstück nicht nur die bei einer Behandlung potentiell mit Körperflüssigkeit in Kontakt kommenden Teile der Vorrichtung sondern auch Bauteile des Antriebsmechanismus abgenommen werden können, die einem Verschleiß unterliegen. Der Antriebsmechanismus stellt nicht nur eine Antriebsbewegung zum Bewegen der Nadel beim Tätowieren bzw. beim Aufbringen des permanten Make-ups zur Verfügung, sondern koppelt die Antriebsbewegung auf den Nadelschaft. Wegen der hochfrequenten Bewegung der Nadel beim Aufbringen des Farbstoffs unterliegen Bauteile des Antriebsmechanismus einem erheblichen Verschleiß. Die Austauschbarkeit von Bauteilen des Antriebsmechanismus mit Hilfe des Einwegmoduls ermöglicht es, die Nutzungsanzahl und somit die Lebensdauer des Handstücks zu verlängern. Der Austausch von Teilen des Antriebsmechanismus im Zusammenhang mit dem Einwegmodul ermöglicht es darüber hinaus, daß die ausgetauschten Bauteile des Antriebsmechanismus aus einfacheren und kostengünstigeren Werkstoffen gefertigt werden, da sie nur für eine begrenzte Nutzungsdauer vorgesehen sind.

Die Erfindung sieht vor, daß die Kopplungsmittel einen Wandlungsmechanismus zum Umwandeln einer vom Antrieb erzeugten, drehenden Antriebsbewegung in eine in den Nadelschaft eingeleitete Linearbewegung aufweisen. Antriebe sind in Form von Motoren zum Erzeugen von Drehbewegungen in einfachen und kostengünstigen Ausführungen verfügbar.

Ein die Drehbewegung des Antriebs zuverlässig und mit Hilfe verschleißfester Kopplungsmittel die Drehbewegung in eine Linearbewegung umsetzender Mechanismus ist bei einer vorteilhaften Weiterbildung der Erfindung dadurch erreicht, daß der Wandlungsmechanismus eine an den Motor befestigte Taumelscheibe und einen Stößel umfassen, der auf einer beim Betrieb des Antriebs taumelnden Oberfläche der Taumelscheibe läuft und an den Nadelschaft gekoppelt ist.

Eine vorteilhafte Ausgestaltung der Erfindung sieht vor, daß in dem Einwegmodul ein von dem Wandlungsmechanismus umfaßtes Verdrehsicherungsbauteil angeordnet und zusammen mit dem Einwegmodul von dem Handstück abnehmbar ist. Das Verdrehsicherungsbauteil dient zum Verhindern einer Drehbewegung eines oder mehrerer des Wadlungsmechanismuses Bauteile beim Antreiben der Nadel.

Die Erfindung wird im folgenden anhand eines Ausführungsbeispiels unter Bezugnahme auf eine Zeichnung näher erläutert. Hierbei zeigen:
- Figur 1: eine schematische Darstellung einer Vorrichtung zum Aufbringen einer Tätowierung oder von permanentem Make-up teilweise im Schnitt; und
- Figur 2: eine schematische Darstellung eines weiteren Antriebsmechanismus für die Vorrichtung nach Figur 1.

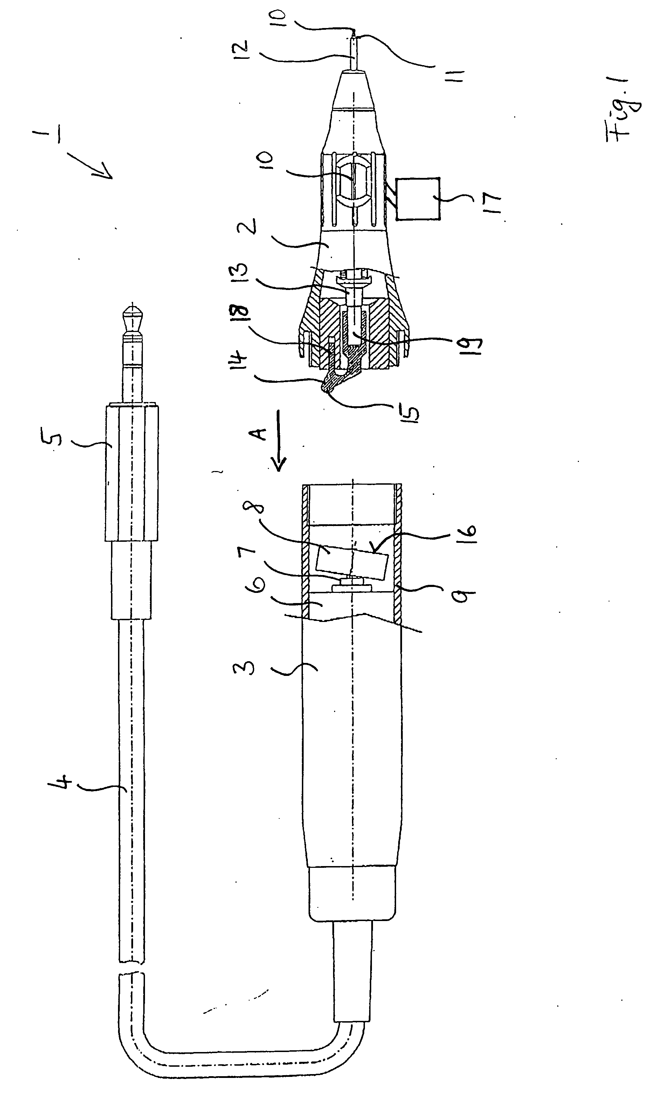

Figur 1 zeigt eine teilweise Schnittdarstellung einer Vorrichtung 1 zum Aufbringen einer Tätowierung oder von permantem Make-up mit einem Einwegmodul 2 und einem Handstück 3. Das Handstück 3 ist mit Hilfe einer hieran angeschlossenen Steuerleitung 4 und einem daran gekoppelten Stecker 5 an ein Steuergerät (nicht dargestellt) anschließbar, um eine geregelte Energieversorgung eines Motors 6 in dem Handstück 3 zu gewährleisten. Auf eine Antriebswelle 7 des Motors 6 ist eine Taumelscheibe 8 montiert, die beim Drehen der Antriebswelle 7 eine taumelnde Bewegung ausführt. Der Motor 6 und die Taumelscheibe 8 sind in einem Gehäuse 9 des Handstücks 3 aufgenommen.

Das Einwegmodul 2 ist getrennt von dem Handstück 3 in Figur 1 dargestellt, da das Einwegmodul 2 als ganzes von dem Handstück 3 abnehmbar ist. Hierbei werden zusammen mit dem Einwegmodul 2 eine Nadel 10, bei der es sich auch eine Gruppe einzelner Nadeln handeln kann, die durch eine Nadeldüsenöffnung 11 greift und in einer auch als Nadelführung dienenden Nadeldüse 12 gelagert ist, ein Nadelschaft 13, der ein Ende der Nadel 10 aufnimmt, sowie ein Stößel 14 von dem Handstück 3 abgenommen. Der Nadelschaft 13 und der Stößel 14 können einteilig ausgebildet sein, so daß der Nadelschaft 13 bei einer solchen Ausführung nicht selbständig als Einzelteil lösbar an einen Antriebsmechanismus gekoppelt und in diesen integriert ist.

Das Einwegmodul 2 wird zum Betreiben der Vorrichtung 1 mit Hilfe einer Steck- und/oder Schraubverbindung an dem Handstück 3 so montiert, daß ein dem Motor 6 zugewandtes Ende 15 des Stößels 14 auf einer Oberfläche 16 der Taumelscheibe 8 aufliegt. Zum Montieren des Einwegmoduls 2 an dem Handstück 3 wird das Einwegmodul 2 gemäß Pfeil A in Richtung des Handstücks 3 bewegt und dann angekoppelt. Bei einer Drehbewegung der Antriebswelle 7 des Motors 6 wird dann das Ende 15 des Stößels 14 durch die Taumelbewegung der Taumelscheibe 8 vor und zurück bewegt. An den Stößel 14 ist ein Nadelschaftendteil 19 der Nadelschaft 13 so gekoppelt, daß die Vor- und Zurückbewegung des Stößels 14 auf die Nadel 10 übertragen wird, die dann sich durch die Nadeldüsenöffnung 11 der Nadeldüse 12 bewegt. Das Ende 15 des Stößels 14 wird mit Hilfe geeigneter Rückhaltemittel (nicht dargestellt) gegen die Oberfläche 16 der Taumelscheibe 8 gedrückt. Hierzu kann beispielsweise eine mechanische Feder genutzt werden, die zweckmäßig gegen den Nadelschaft 13 drückt.

Beim Vor- und Zurückbewegen der Nadel 10 wird ein Farbstoff aus einem in Figur 1 schematisch dargestellten Farbstoff-Vorratsbehälter 17 ausgebracht, der mit der Nadeldüsenöffnung 11 in Verbindung steht. Die Art und Weise, wie der Farbstoff ausgebracht wird, beispielsweise mit oder ohne Druckbeaufschlagung, ist für die Erfindung nicht kritisch. Es können die verschiedenen als solche bekannten Arten des Farbaustrags genutzt werden. Der Farbstoff-Vorratsbehälter 17 kann beispielsweise in Form eines in dem Einwegmodul 2 zur Verfügung stehenden Hohlraums in das Einwegmodul 2 integriert oder als Farbpatrone zum Austauschen lösbar an dem Einwegmodul 2 montiert sein. Darüber hinaus kann auf ein Farbstoff-Vorratsbehälter 17 ganz verzichtet werden. So kann auch mittels Eintauchens der Nadeldüse 12 in einen Farbstoffvorrat gearbeitet werden. Hierbei gelangt der auszubringende Farbstoff mittels Kapillarwirkung in die Nadeldüse 12 bis in ein Farbstoff-Reservoir, welches bei einer Ausführungsform vorgesehen sein kann, und wird dann bei der Nutzung aufgrund der Bewegung der Nadel 10 durch die Nadeldüsenöffnung 11 ausgebracht.

Mit Hilfe der in Figure 1 dargestellten Ausgestaltung der Vorrichtung 1 ist es möglich, zumindest den Stößel 14, welcher mit einer Verdrehsicheung 18 versehen ist, als Teil eines Antriebsmechanismus zum Bewegen der Nadel 10 beim Tätowieren oder beim Aufbringen von permanentem Make-up zusammen mit den Bauteilen vom Handstück 3 gemeinsam zu trennen, die potentiell mit Körperflüssigkeit des Kunden infiziert werden. Hiervon betroffen sind in der Regel die Nadel 10, die Nadeldüse 12 und möglicherweise der Nadelschaft 13.

Die in Figur 1 dargestellte Ausführungsform des Antriebsmechanismus für die Nadelbewegung, welcher die Taumelscheibe 8 und den Stößel 14 umfaßt, ist nur eine mögliche Ausführungsform für solche Vorrichtungen zum Tätowieren oder zum Aufbringen von permanentem Make-up. Dem Fachmann sind weitere Antriebsmechanismen bekannt. Auch bei anderen bekannten Antriebsmechanismen kann der Erfindungsgedanke, Bauteile des Antriebsmechanismus in das Einwegmodul zu integrieren, implementiert werden, um insbesondere Bauteile zusammen mit dem Einwegmodul auszutauschen, die bei der Nutzung der Vorrichtung einem erhöhten Verschleiß unterliegen. Die im Zusammenhang mit der Erfindung erläuterten Vorteile ergeben sich dann entsprechend.

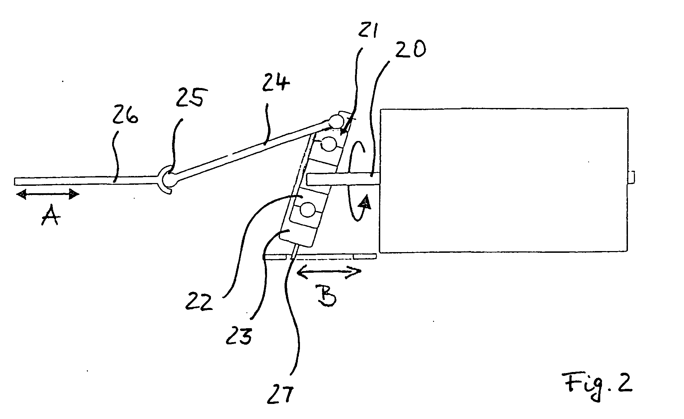

Als Beispiel zeigt Figur 2 eine schematische Darstellung eines weiteren Antriebsmechanismus für die Vorrichtung 1 nach Figur 1, bei dem auf einer Antriebswelle 20 ein Kugellager 21 mit einem feststehenden und schräg auf der Antriebswelle 20 montierten Innenring 22 und einem freilaufenden Außenring 23 montiert ist. An dem Außenring 23 wird eine Pleuelstange 24 gehalten, die über ein Gelenk 25 mit einer Schubstange 26 gekoppelt ist. Beim Drehen der Antriebswelle 20 führt das Kugellager 21 eine taumelnde Bewegung aus, welche mit Hilfe der Pleuelstange 24 und der Schubstange 26 in eine lineare Antriebsbewegung für den Nadelschaft in Pfeilrichtung A umgesetzt wird. Hierbei wird der Außenring 23 mit Hilfe einer Verdrehsicherung 27 festgehalten, die sich in Pfeilrichtung B vor und zurück bewegt.

Eine zweckmäßige Ausführungsform sieht vor, die Taumelscheibe 8 bei der Vorrichtung 1 nach Figur 1 durch das Kugellager 21 mit dem Innenring 22 und dem freilaufenden Außenring 23 nach Figur 2 zu ersetzen. Hierbei wird ein Verdrehen des Außenrings 23 dann zweckmäßig mittels Klemmens des Außenrings 23 beim Anbringen des Einwegmoduls 2 an dem Handstück 3 verhindert. Alternativ oder ergänzend hierzu kann an dem Einwegmodul 2 eine Ausnehmung (nicht dargestellt) vorgesehen sein, in welche der Außenring 23 oder ein hieran gebildeter Vorsprung, vergleichbar zur der Verdrehsicherung 27 (vgl. Figur 2), eingreift. Die Ausnehmung kann vorteilhaft am Gehäuse des Einwegmoduls 2 gebildet sein.

## Patentansprüche

1. Vorrichtung zum Aufbringen einer Tätowierung oder von permanentem Make-up, mit einem Einwegmodul(2), welches lösbar an einem Handstück (3) angeordnet ist, einer Nadel (10), die mit einem Ende in einem Nadelschaft (13) gehalten wird, die bewegbar in einer an dem Einwegmodul (2) gebildeten Nadelführung gelagert ist und die mit einem anderen Ende durch eine Nadeldüsenöffnung (11) zum Ausbringen eines Farbstoffs verläuft, und einem an den Nadelschaft (13) zum Vor- und Zurückbewegen der Nadel (10) gekoppelten und von mehreren Bauteilen (6, 7, 8, 14, 19) gebildeten Antriebsmechanismus, wobei die mehreren Bauteile (6, 7, 8, 14, 19) des Antriebsmechanismus einen Antrieb (6, 7) und Kopplungsmittel (8, 14, 19) zum Koppeln des Antriebs (6, 7) an den Nadelschaft (13) umfassen und zumindest ein Teil der mehreren Bauteile (14, 19) des Antriebsmechanismus in dem Einwegmodul (2) angeordnet ist und zusammen mit dem Einwegmodul (2) von dem Handstück (3) abnehmbar ist, **dadurch gekennzeichnet, daß** die Kopplungsmittel (8, 14, 19) einen Wandlungsmechanismus (8, 14) zum Umwandeln einer von den Antrieb (6, 7) erzeugten, drehenden Antriebsbewegung in eine in den Nadelschaft (13) eingeleitete Linearbewegung aufweisen und daß der Wandlungsmechanismus (14) teilweise in dem Einwegmodul (2) gebildet und zusammen mit dem Einwegmodul (2) von dem Handstück (3) abnehmbar ist.

2. Vorrichtung nach einem der vorangehenden Ansprüche, **dadurch gekennzeichnet, daß** der Wandlungsmechanismus eine an dem Motor befestigte Taumelscheibe(8) und einen Stößel (14) umfassen, der auf einer beim Betrieb des Antriebs (6, 7) taumelnden Oberfläche (16) der Taumelscheibe (8) läuft und an den Nadelschaft (13) gekoppelt ist.

3. Vorrichtung nach einem der vorangehenden Ansprüche, **dadurch gekennzeichnet, daß** in dem Einwegmoduls (2) ein von dem Wandlungsmechanismus umfaßtes Verdrehsicherungsbauteil (18) angeordnet und zusammen mit dem Einwegmodul (2) von dem Handstück (3) abnehmbar ist.

4. Einwegmodul (2) zum lösbaren Ankoppeln an ein Handstück (3) eines Tätowier- und/oder Permanent-Make-up-Handgeräts (1) mit einer Nadel (10), die vor und zurück bewegbar in einer Nadelführung gelagert ist, die mit einem Ende in einem Nadelschaft (13) gehalten wird und die mit einem anderen Ende durch eine Nadeldüsenöffnung (11) zum Ausbringen eines Farbstoffs verläuft, **gekennzeichnet durch** wenigstens ein mit dem Nadelschaft (13) gekoppeltes Bauteil (14) eines Antriebsmechanismus zum Antreiben der Nadel (10) in der Nadelführung und **durch** die Nadeldüsenöffnung(11), wobei das wenigstens eine Bauteil (14) ein Bauteil eines Wandlungsmechanismus zum Wandeln einer drehenden Antriebsbewegung in eine in den Nadelschaft (13) einzuleitende Linearbewegung ist.

## Claims

1. Tattooing or permanent make-up device, with a disposable module (2) which is arranged releasably on a handpiece (3), with a needle (10) which is held at one end in a needle shaft (13), is mounted movably in a needle guide formed on the disposable module (2) and, at another end, extends through a needle nozzle orifice (11) for discharge of a dye, and with a drive mechanism which is made up of several structural members (6, 7, 8, 14, 19) and is coupled to the needle shaft (13) in order to achieve reciprocating movements of the needle (10), the several structural members (6, 7, 8, 14, 19) of the drive mechanism comprising a drive means (6, 7) and coupling means (8, 14, 19) for coupling the drive means (6, 7) to the needle shaft (13), and at least part of the several structural members (14, 19) of the drive mechanism being arranged in the disposable module (2) and being able to be removed from the handpiece (3) together with the disposable module (2), **characterized in that** the coupling means (8, 14, 19) comprise a conversion mechanism (8, 14) for converting a rotary drive motion, generated by the drive means (6, 7), into a linear motion introduced into the needle shaft (13), and **in that** the conversion mechanism (14) is formed partly in the disposable module (2) and can be removed from the handpiece (3) together with the disposable module (2).

2. Device according to one of the preceding claims, **characterized in that** the conversion mechanism comprise a wobble plate (8), secured on the motor, and a tappet (14) which runs on a surface (16) of the wobble plate (8) wobbling during operation of the drive means (6, 7) and is coupled to the needle shaft (13).

3. Device according to one of the preceding claims, **characterized in that** an anti-twist component (18) forming part of the conversion mechanism is arranged in the disposable module (2) and can be removed from the handpiece (3) together with the disposable module (2).

4. Disposable module (2) for releasable coupling to a handpiece (3) of a handheld tattooing and/or permanent make-up apparatus (1), with a needle (10) which is mounted in a needle guide so that it can be moved to and fro and is held with one end in a needle shaft (13) and, with another end, extends through a needle nozzle orifice (11) for discharge of a dye, **characterized by** at least one structural member (14) of a drive mechanism coupled to the needle shaft (13) for the purpose of driving the needle (10) in the needle guide and through the needle nozzle orifice (11), the at least one structural member (14) being a structural member of a conversion mechanism for converting a rotary drive motion into a linear motion to be introduced into the needle shaft (13).

## Revendications

1. Dispositif permettant d'appliquer un tatouage ou un maquillage permament, comportant un module à usage unique (2), disposé de façon amovible sur un porte-outil (3), une aiguille (10), retenue par une extrémité dans une tige d'aiguille (13), logée de façon mobile dans un guide d'aiguille formé sur le module à usage unique (2) et s'étendant par une autre extrémité à travers une ouverture d'injecteur à aiguille (11) destinée à déliver un colorant, et un mécanisme d'entraînement couplé à la tige d'aiguille (13) destiné à entraîner l'aiguille (10) dans un mouvement de va-et-vient et formé de nombreux composants (6, 7, 8, 14, 19), les nombreux composants (6, 7, 8, 14, 19) du mécanisme d'entraînement comprenant un entraînement (6, 7) et des moyens de couplage (8, 14, 19) destinés à coupler l'entraînement (6, 7) à la tige d'aiguille (13) et au moins une partie des nombreux composants (14, 19) du mécanisme d'entraînement étant disposée dans le module à usage unique (2) et pouvant être démontée du porte-outil (3) conjointement avec le module à usage unique (2), **caractérisé en ce que** les moyens de couplage (8, 14, 19) présentent un mécanisme de conversion (8, 14) destiné à convertir un mouvement d'entraînement rotatif produit par l'entraînement (6, 7) en un mouvement linéaire induit dans la tige d'aiguille (13) et **en ce que** le mécanisme de conversion (14) est formé en partie dans le module à usage unique (2) et peut être démonté du porte-outil (3) conjointement avec le module à usage unique (2).

2. Dispositif selon la revendication précédente, **caractérisé en ce que** le mécanisme de conversion comprend un plateau oscillant (8) fixé à l'entraînement et un pilon (14), qui fonctionne sur une surface supérieure (16) du plateau oscillant (8) lors de l'actionnement de l'entraînement (6, 7) et **en ce qu'**il est couplé à la tige d'aiguille (13).

3. Dispositif selon l'une quelconque des revendications précédentes, **caractérisé en ce qu'**un composant antipivotement (18) compris dans le mécanisme de conversion est disposé dans le module à usage unique (2) et peut être démonté du porte-outil (3) conjointement avec le module à usage unique (2).

4. Module à usage unique (2) pour couplage amovible sur un porte-outil (3) d'un matériel à main (1) de tatouage et/ou de maquillage permanent comportant une aiguille (10) logée de façon mobile dans un guide d'aiguille pour effectuer un mouvement de va-et-vient, retenue par une extrémité dans une tige d'aiguille (13) et s'étendant par une autre extrémité à travers une ouverture d'injecteur à aiguille (11) destinée à déliver un colorant, **caractérisé par** au moins un composant (14) d'un mécanisme d'entraînement couplé à la tige d'aiguille (13) destiné à entraîner l'aiguille (10) dans le guide d'aiguille et à travers l'ouverture d'injecteur à aiguille (11), où ledit au moins un composant (14) étant un composant d'un mécanisme de conversion destiné à convertir un mouvement d'entraînement rotatif en un mouvement linéaire induit dans la tige d'aiguille (13).
